# EUROPEAN PATENT APPLICATION

(11) **EP 2 574 930 A1**
(43) Date of publication of application: **03.04.2013**
(21) Application number: 12182622.6
(22) Date of filing: 31.08.2012
(51) Int. Cl.: G01N 33/574

(54) **Method and biomarker for evaluating metastasis, and siRNA compound for inhibiting metastasis**

(30) Priority: 01.09.2011 US 201161530003 P
(71) Applicant: National Cheng Kung University, Tainan City 701 (TW)
(72) Inventor: Liao, Pao-Chi, Tainan City 701 (TW); Cheng, Hung-Chi, Tainan City 710 (TW); Chang, Ying-Hwa, Taipei City 100 (TW); Lee, Shu-Hui, Changhua County 503 (TW)
(74) Representative: Leathley, Anna Elisabeth

(57) **Abstract**

A method and biomarker for evaluating metastasis, and an siRNA compound for inhibiting metastasis. The method of the present invention includes: providing a sample of a subject, which includes a normal tissue and a tissue to be detected; detecting expression of a biomarker of the normal tissue and the tissue to be detected, respectively, wherein the biological marker is SERPINA1; and comparing the expression of the biological marker of the normal tissue and the tissue to be detected. When the expression of the biological marker of the tissue to be detected is higher than the normal tissue, it represents that the subject is at a risk of suffering from metastasis.

## Description

### CROSS REFERENCE TO RELATED APPLICATION

This application claims the benefit of filing date of U. S. Provisional Application Serial Number 61/530,003, entitled "FN1, TIMP1, and SERPINA1 Regulate the Migration, Invasion, and Pericellular Fibronectin Assembly of Cancer Cells in Metastasis: A Novel Biological Significance Revealed by Quantitative Proteomics Analysis of NSCLC Cell Secretomes" filed September 1, 2011 under 35 USC § 119(e)(1).

### BACKGROUND OF THE INVENTION

### 1. Field of the Invention

The present invention relates to a method and a biomarker for evaluating metastasis, and an siRNA compound for inhibiting metastasis. More specifically, the present invention relates to a method and a biomarker for evaluating an incidence rate of metastasis, and an siRNA compound for inhibiting metastasis derived therefrom.

### 2. Description of Related Art

Lung cancer is a major factor causing death around the world, and 90% of lung cancer patients die due to metastasis. Metastasis is the spread of tumor cells from lung to other organs. Once the metastasis occurs, the symptoms of cancers are harder to control and cure. In addition, metastasis involves complicated mechanisms, wherein the tumor cells with invasive or migration capacities separate from primary organs, invade to peripheral blood vessel or lymph capillary, and spread to other non-adjacent organs via blood circulation or lymphatic system to form a secondary cancer.

In addition, there are several types of lung cancer including small cell lung cancer (SCLC) and non-small cell lung cancer (NSCLC). Recently, most lung cancer patients suffer from NSCLC. NSCLC is divided into three sub-types including adenocarcinoma, squamous cell lung carcinoma and large cell lung carcinoma. 40% of lung cancers are adenocarcinoma, 30% thereof are squamous cell lung carcinoma, and 9% thereof are large cell carcinoma.

Some studies indicate that tumor cells have to interact with specific molecules or factors in the secretome during metastasis, and some of the specific molecules or factors are regulated by secretory factors with catalytic or recognition properties. Hence, it may be helpful to prevent and diagnose metastasis if the regulation related to the secretome can be analyzed, the relations between each proteins and cancers can be identified by several experimental manners, important proteins highly related to metastasis can be selected from the secretome, and the relation between the selected proteins and cancers can be well-understood.

Since the secretome is highly related to cancer metastasis, it is desirable to identify proteins related to cancer metastasis through an effective method for analyzing the secretome, and then the identified proteins can be used to evaluate metastasis or develop drugs for inhibiting metastasis before secondary cancers form or at early stages of metastases. Hence, the metastasis can be prevented or found in early stages, so the incidence rate of metastasis can be reduced and the mortality from cancers can further be decreased.

### SUMMARY OF THE INVENTION

An object of the present invention is to provide a method for evaluating metastasis, which can be used to evaluate whether a subject is at a risk of suffering from metastasis.

Another object of the present invention is to provide a biomarker for evaluating metastasis, which can be used to evaluate an incidence rate of metastasis in a subject. In addition, the biomarker for evaluating metastasis can further be used as a target in a gene therapy or used in a protein drug development.

A further object of the present invention is to provide an siRNA compound for inhibiting lung metastasis, which can be used in a gene therapy of lung cancer treatment. To achieve the object, the method for evaluating metastasis of the present invention comprises the following steps: (A) providing a sample of a subject including a normal tissue and a tissue to be detected; (B) detecting expression of a biomarker in the normal tissue and the tissue to be detected respectively, wherein the biomarker is serpin peptidase inhibitor, clade A α1-antitrypsin (SERPINA1); and (C) comparing the expression of the biomarker in the normal tissue with that in the tissue to be detected, wherein when the expression of the biomarker in the normal tissue is higher than that in the tissue to be detected, this indicates that the subject is at a risk of suffering from metastasis. Herein, the normal tissue can be a normal organ tissue or a normal blood tissue, and the tissue to be detected can be a tumor tissue or a blood tissue from a subject with lung cancer.

In addition, the biomarker for evaluating metastasis, is at least one selected from a group consisting of a nucleotide sequence, a complementary sequence of the nucleotide sequence, a derivative of the nucleotide sequence, an amino-acid sequence, a derivative of the amino-acid sequence, a fragment of the amino-acid sequence, a mutation of the amino-acid sequence, and an antibody corresponding to the amino-acid sequence of SERPINA1. Preferably, the biomarker is at least one selected from the group consisting of the amino-acid sequence, the derivative of the amino-acid sequence, the fragment of the amino-acid sequence, the mutation of the amino-acid sequence, and the antibody corresponding to the amino-acid sequence of SERPINA1. The nucleotide sequence preferably encodes SERPINA1. The amino acid sequence is preferably that of SERPINA1. The antibody preferably binds to the amino acid sequence of SERPINA1. The binding is preferably specific and/or selective.

The biomarker is for use in diagnosis, e.g. for use in evaluating metastasis.

Although it is known that SERPINA1 is a type of serine protease inhibitor, the role of SERPINA1 is still unidentified. Some studies indicate that the expression of SERPINA1 in serum of cancer patients is much higher than that in healthy people. The present inventors found that SERPINA1 participates in several cancer metastases, including ovarian cancer, cervical cancer, colorectal cancer, breast cancer and lung cancer (especially, non-small lung cancer (NSCLC)). In addition, the present inventors also found that SERPINA1 participates in regulation of the invasive and migration capacities of lung tumor cells such as CL1-5, and regulates the aggregation of fibronectin (FN1) on surfaces of tumor cells, wherein the aggregation ofFN1 may increase the probability of metastasis. Therefore, the present invention uses SERPINA1 as a biomarker for evaluating metastasis, based on the aforementioned findings.

In the method for evaluating metastasis, the lung cancer can be small cell lung cancer (SCLC) or non-small cell lung cancer (NSCLC). Preferably, the lung cancer is NSCLC.

In addition, the biomarker used in the present invention may have a nucleotide sequence with 50% or more identity to a sequence represented by SEQ ID NO: 1. Preferably, the biomarker of the present invention has a nucleotide sequence with 70-100% identity to a sequence represented by SEQ ID NO: 1. More preferably, the biomarker of the present invention has a nucleotide sequence with 90-100% identity to a sequence represented by SEQ ID NO: 1. In addition, the biomarker used in the present invention may have an amino-acid sequence with 50% or more identity to a sequence represented by SEQ ID NO: 2. Preferably, the biomarker of the present invention has an amino-acid sequence with 70-100% identity to a sequence represented by SEQ ID NO: 2. More preferably, the biomarker of the present invention has an amino-acid sequence with 90-100% identity to a sequence represented by SEQ ID NO: 2. In the present invention, the term "identity" refers to the percentage of identical components, i.e. the percentage of identical nucleotides or amino-acid residues in the nucleotide sequence or the amino-acid sequence.

The step (B) of the method for evaluating metastasis of the present invention may be: detecting the expression of mRNA, protein, protein derivatives, or protein fragments of SERPINA1 in the normal tissue or the tissue to be detected respectively. Preferably, the step (B) is: detecting the expression of protein of SERPINA1 in the normal tissue or the tissue to be detected respectively.

Any techniques generally known in the art can be used in the method for evaluating metastasis of the present invention to identify the expression of SERPINA1 in the normal tissue or the tissue to be detected, for example, western blot analysis, electrophoresis, enzyme-linked immunosorbent assay (ELISA), immunohistochemistry (IHC), immunoprecipitation (IP), mass spectrometry (MS), real time polymerase chain reaction (RT-PCR) or real time quantitative polymerase chain reaction (real time Q-PCR). Preferably, the expression of protein of SERPINA1 in the normal tissue or the tissue to be detected are detected respectively through western blot analysis, electrophoresis, enzyme-linked immunosorbent assay (ELISA), immunohistochemistry (IHC), immunoprecipitation (IP) or mass spectrometry (MS).

Since the secretome is highly related to metastasis, SERPINA1 in the secretome has potential for evaluating metastasis. Hence, the method of the present invention is performed by detecting the expression of SERPINA1 in a sample from a subject to evaluate the risk or the incidence rate of metastasis. Therefore, it is possible to find and exactly estimate the metastatic tumor cells before a secondary cancer is formed or at an early stage of the secondary cancer, so the survival rate of lung cancer patients can further be increased. Furthermore, the biomarker of the present invention may further be used in metastasis predictions or as prognostic indicators in clinic, and applied to targeting therapy.

Furthermore, the present invention also provides an siRNA compound for inhibiting lung metastasis, which comprises: a target sequence, which is selected from genes of SERPINA1. The siRNA compound of the present invention can be used in RNA interference (RNAi) gene therapy to inhibit the invasion and migration of lung cancer, so the effect of cancer treatment can be improved and the mortality from cancers can be decreased. The siRNA compound is thus for use in therapy, preferably for use in inhibiting lung metastasis.

In the siRNA compound for inhibiting lung metastasis of the present invention, the target sequence may comprise 20-25 continuous nucleotides from SERPINA1. Preferably, the target sequence comprises 20-25 continuous nucleotides from SERPINA1 represented by SEQ ID NO: 1.

In all cases the subject is preferably human.

Other objects, advantages, and novel features of the invention will become more apparent from the following detailed description when taken in conjunction with the accompanying drawings.

### BRIEF DESCRIPTION OF THE DRAWINGS

FIG. 1 is a result of western blotting analysis of SERPINA1 of the present invention;
FIG. 2 is a result of wound-healing assay of the present invention;
FIG. 3 is a result of migration assay of the present invention;
FIG. 4 is a result of matrigel invasion assay of the present invention;
FIG. 5 is a result of flow cytometry of CL1-0 cells of the present invention;
FIG. 6 is a result of flow cytometry of CL1-5 cells of the present invention;
FIG. 7 is a result of flow cytometry of a control group of the present invention;
FIG. 8 is a result of flow cytometry of an experimental group of the present invention; and
FIG. 9 is a result of lung colony assay of the present invention.

### DETAILED DESCRIPTION OF THE PREFERRED EMBODIMENT

### Lung cancer cell line CL1

In the present embodiment, lung cancer cell lines (CL1-0 and CL1-5 cells) with different invasive and metastatic capabilities were provided by Department of Internal Medicine, National Taiwan University Hospital, Taipei, Taiwan, Republic of China. The cells were maintained in an RPMI 1640 medium supplemented with 10% fetal bovine serum (FBS) and antibiotics at 37 °C under 5%CO₂.

### Harvest of conditioned media from lung cancer cell lines

CL 1 cells were grown to confluence in tissue culture dishes, washed with serum-free media three times to avoid serum contamination, and incubated in serum-free media for 24 h. The supernatants of the conditioned media (CM) were then harvested and centrifuged to eliminate the intact cells and contaminants. Next, the supernatants were concentrated and desalted by centrifugation in Amicon Ultra-15 tubes (molecular weight cutoff 3000 Da; Millipore, Billerica, MA). The protein concentrations of CL1 CM samples were determined using the Bradford protein assay reagent (Biorad). Herein, the protein concentrations obtained by Bradford protein assay reagent were the concentrations of secretome.

### Separation and purification of secretome

The obtained secretome from concentrated CM samples was purified with a purification gel. Herein, the purification gel was prepared as follows. First, 0.6 mL of H₂O, 2.22 mL of 1.5 M Tris-HCl [pH 8.8], 90 µL of 10% SDS, 6 mL of Bis/Acrylamide, 90 µL of 10% ammonium persulfate, and 5 µL of TEMED were mixed well and set to polymerize for 1 hour to obtain a resolving gel portion (i.e. high-density layer). Then, 2.9 mL of H₂O, 0.5 mL of 1 M Tris-HCl [pH 6.8], 40 µL of 10% SDS, 520 µL of Bis/Acrylamide, 40 µL of 10% ammonium persulfate, and 4 µL of TEMED were mixed and poured on the resolving gel portion. After a setting process was completed, a stacking gel portion (i.e. low-density layer) was obtained. After the aforementioned process, a purification gel was obtained, which comprises a resolving gel portion (i.e. high-density layer) and a stacking gel portion (i.e. low-density layer). In addition, the low-density layer was stacked on the high-density layer.

A total of 100 µg of secretome was mixed with 13 µL of H₂O,5 µL of 4X SDS sample buffer, and 2 µL of 0.5M DTT and then boiled under 95 °C for 10 min. The purification was run at 55 V The electrophoresis was stopped after the sample had just passed into the resolving gel portion, and the gels were then stained using Coomassie Brilliant Blue (CBB) R-250.

### In-gel digestion

The secretome sample located on an interface between the low-density layer and the high-density layer was collected and the gel pieces were diced into about 1 mm³. Gel slices were washed and dehydrated three times in 25 mM ammonium bicarbonate (ABC) (pH 7.9) and 50mM ABC/50% acetonitrile. A protein reduction was subsequently performed by incubating 0.5M DTT for 1 h at 56 °C and then alkylating with 50 µL saturated IAA for 45 min at room temperature in the dark (i.e. carbamidomethylation process). After two subsequent wash/dehydration cycles, each gel sample was digested with 4 µg (1:25, w/w) of sequencing-grade modified trypsin (Promega)/25 mM ammonium bicarbonate and incubated at 37 °C for an overnight digestion (16-18 hours). After the digestion process, peptides, which were obtained from the secretome, were extracted twice in 100 µL of 50 % ACN in 5 % formic acid. The extracted peptides were enriched using OMIX C18 pipet tips (Varian) to remove any contaminants, which may have affected the signal of the sequential iTRAQ labeling.

### Isotope labeling of peptides from secretome

The enriched peptides from the secretome were labeled with the iTRAQ reagent (Applied Biosystems, Foster City, CA, USA) according to the manufacturer's protocol.

Briefly, one unit of iTRAQ reagent was thawed and reconstituted in ethanol (70 µL), wherein one unit was defined as the amount of reagent required to record 100 µg of protein. The obtained peptide mixtures were reconstituted with 20 µL of iTRAQ dissolution buffer. 70 µL iTRAQ reagent solutions (iTRAQ115: iTRAQ116 = 1:1, or iTRAQ114: iTRAQ117 = 1:1) were combined with the peptide mixtures from the secretomes. The extracted peptide mixtures were then pooled and dried by vacuum centrifugation. The dried peptide mixture was reconstituted and acidified with 10 µL of buffer (5 mM K₂HPO₄ and 25% ACN [pH 3]) for fractionation by SCX chromatography using an AKTA FPLC system (GE Healthcare) to reduce the complication of the samples. A total of 28 fractionations were generated and were desalted using OMIX C18 pipet tips (Varian) according to the user instructions in order to remove the salts which may influence the signal of isotope reagents.

### Analysis of peptides from secretome with LC-ESI-MS/MS

iTRAQ-labeled samples were reconstituted in eluent buffer A (0.1% (v/v) FA in H2O) and analyzed by LCMS/MS. The buffer B (0.1 % (v/v) FA in ACN) gradient started from 0% to 5% at 2 mins and then progressed to 37% in 140 mins. Peptides were eluted at 200-300 nL/min.

Peptide fragmentation by collision-induced dissociation was performed automatically using the information-dependent acquisition in Analyst QS v1.1 (Applied Biosystems). The method applied a 1-s TOF MS scan and automatically switched to three 2-s product ion scans (MS/MS) when a target ion reached an intensity of greater than 20 counts. TOF MS scanning was undertaken over the range 400-2000 m/z. Product ion scans were undertaken over the range 100-2000 m/z at low resolution.

### Database comparison

The results from LC-MS/MS were batch-searched against the Swiss-Prot human sequence database (version 20090616; 468851 sequences) using the MASCOT algorithm (v2.1.0, Matrix Science, London, U.K.). The peak list in the MS/MS spectra generated under ESI-Q-TOF was extracted with AnalystQS 1.1 (Applied Biosystems) with the default charge state set to 2+, 3+, and 4+. The MS and MS/MS centroid parameters were set to 10% height percentage and to a merge distance of 0.1 amu. For the MS/MS grouping, the averaging parameters consisted of rejection of spectra with less than five peaks or precursor ions with less than 10 counts/s. Search parameters for peptide and for MS/MS mass tolerance were 1 and 0.5 Da, respectively, with allowance for two missed cleavages made in the trypsin digest and for variable modifications of deamidation (Asn, Gln), oxidation (Met), iTRAQ (Nterminal), iTRAQ (Lys), and carboxyamidomethylation (Cys). Peptides were considered to have been identified if their MASCOT individual ion score was higher than the MASCOT score 20.

After the aforementioned analysis, 331 proteins were identified from the secretome of lung cancer samples.

### Protein quantification

For protein quantification, data analysis for the iTRAQ experiments was performed with the software Multi-Q. The raw data files from QSTAR Pulsar I were converted into files of mzXML format by the program mzFAST, and the search results in MASCOT were exported in comma-separated-values (CSV) data format. After the data conversions, Multi-Q selected iTRAQ labeled peptides with confident MS/MS identifications (MASCOT score 20), detected signature ions (m/z 114, 115, 116, and 117), and performed an automated quantification of peptide abundance.

To calculate the average protein ratios, the ratios of quantified, unique iTRAQ peptides were weighted according to their peak intensities to minimize standard deviation.

### Bioinformatics analysis

The identified proteins were analyzed using the SignalP, SecretomeP, and TMHMM programs to predict the possibility of protein secretion through classic or through nonclassic secretion pathways and the presence of transmembrane domains in the protein sequence. The molecular functions of the identified proteins were determined based on a search against the Human Protein Reference Database (HPRD) (http://www.hprd.org/).

After the bioinformatics analysis, more than 77.3% of identified proteins may be assumed to be secreted proteins through different secretion pathways. In addition, 66 proteins were identified through the bioinformatics analysis, which have significant expression differences in CL1-0 and CL1-5 and may be related to lung metastasis.

### Statistical analysis

All experiments were performed in triplicate, and the results are shown as the mean ± SD. The nonparametric Mann-Whitney U test was employed to analyze the comparison between two groups. P values less than 0.05 were considered statistically significant.

### Western blotting analysis

12 proteins are selected from the identified 66 proteins related to lung metastasis, which includes Nidogen-1, MAGE-A4, PRDX1, CKB, PLAU, SERPINA1, TIMP, FN1, HSPA5, COL6A1, THBS1, and CTSL1. These 12 proteins were examined through western blotting analysis, in order to identify whether these proteins were indeed related to lung metastasis.

First, 5-30 µg of secreted proteins from the CL1 cell CMs were separated on a 12% SDS-PAGE and transferred to PVDF membranes (Millipore). The membranes were blocked in a 5% nonfat milk solution for 1 hour at room temperature and then probed with various antibodies against the selected proteins (Santa Cruz Biotechnology) and against anti-α-tubulin (Calbiochem) for 3 hours. The membranes were washed with TBST 3 times and incubated with horseradish peroxidase-conjugated secondary antibodies at a dilution of 1:5000 at room temperature for 1 hr. The membranes were washed with TBST 5 times before developing them with enhanced chemiluminescence detection.

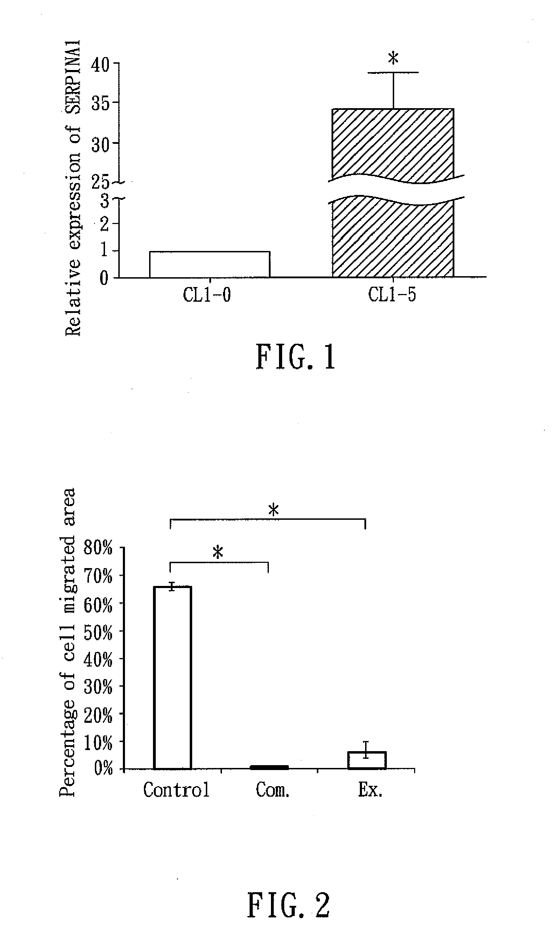

The results of western blotting analysis show that the expression of PLAU, SERPINA1, TIMP, FN1, HSPA5, COL6A1, THBS1 and CTSL1 can be identified in the CL1-5 with high invasive capacity, and the expression of Nidogen-1, MAGE-A4, PRDX1 and CKB can be identified in the CL1-0 with low invasive capacity. In addition, FIG. 1 shows the experimental result about SERPINA1. As shown in FIG. 1, the expression of SERPINA1 in CL1-5 is much higher than that in CL1-0, and shows significant differences. Hence, according to the results of western blotting analysis, the proteins highly related to lung metastasis, especially SERPINA1 of the present invention can be identified through the aforementioned gel purification, isotope labeling, and mass spectrometry of the present embodiment.

### siRNA interference

There are no studies showing that SERPINA1 is related to metastasis. Herein, SERPINA1 gene silencing was performed to identify the relation between SERPINA1 and metastasis.

In the present analysis, SERPINA1 siRNA was provided, which was a mixture containing three DNA sequence sets represented by a set consisting of SEQ ID NO: 3 and SEQ ID NO: 4, a set consisting of SEQ ID NO: 5 and SEQ ID NO: 6, and a set consisting of SEQ ID NO: 7 and SEQ ID NO: 8. In addition, FN1 siRNA was also provided, which was a mixture containing three DNA sequence sets represented by a set consisting of SEQ ID NO: 9 and SEQ ID NO: 10, a set consisting of SEQ ID NO: 11 and SEQ ID NO: 12, and a set consisting of SEQ ID NO: 13 and SEQ ID NO: 14. Then, CL1-5 cells were transfected with the aforementioned siRNAs using the siRNA transfection reagent according to the manufacturer's instructions (Santa Cruz Biotechnology, Santa Cruz, CA). For each transfection, 80 pmol of the SERPINA1 siRNA, FN1 siRNA or control siRNA (scrambled siRNA) with 6 µL of siRNA transfection reagent was added to 100 µL of siRNA transfection media. The solution was mixed gently and overlaid onto the CL1-5 cells for 24 h. The media was then aspirated and 3x10⁵ CL1-5 cells were grown in 2 mL of RPMI 1640 containing 10% fetal bovine serum (FBS) on six-well culture dishes reaching 80% confluence at 37 °C under 5%CO2_{.} Herein, the experimental group (Ex.) was CL1-5 cells transfected with SERPINA1 siRNA, the control group (Control) was CL1-5 cells transfected with scrambled siRNA, and the comparative group (Comp.) was CL1-5 cells transfected with FN1 siRNA. Then, the aforementioned western blotting analysis was performed to identify the results of siRNA interference. In addition, the CL1-5 cells transfected with the aforementioned siRNA were further used to perform the following wound healing assay, migration assay and matrigel invasion assay.

The results of siRNA interference show that the transfection of SERPINA1 siRNA can inhibit the protein expression of SERPINA1 in CL1-5 cells, and the transfection ofFN1 siRNA also can inhibit the protein expression of FN1 in CL1-5 cells. Hence, the SERPINA1 siRNAs used in the present embodiment has an effect on inhibiting the expression of SERPINA1 protein.

### Wound-healing assay

Cell migration ability was examined with the commercial ibidi Culture-Insert (Applied BioPhysics, Inc., Troy, New York, USA). Cells were seeded on the insert for 12 hrs, and the inserts were removed. Photographs were taken at 0 hr and 24 hrs at the same position in the cell-free gap insert with 100X magnification. The Image-Pro Plus 6.0 software was used to calculate the cell migrating area (Media Cybernetics, Inc. Bethesda, MD, USA).

The results show that SERPINA1 siRNA led to a dramatic decrease of invasion in the SERPINA1-siRNA-transfected CL1-5 cells in comparison with the scrambled-siRNA-transfected CL1-5 cells. These results demonstrate that the knock-down of SERPINA1 expression impairs migration and invasion in CL1-5 cells and that SERPINA1 is critical for migration and invasion in CL1-5 cells. In addition, FN1 siRNA also led to a dramatic decrease of invasion in the FN1-siRNA-transfected CL1-5 cells, as shown in FIG. 2.

### Migration assay

A transwell membrane (8-µm pore size, BD Biosciences) was used for a transwell migration assay, The CL1 cells were trypsinized, washed, and kept suspended in their medium without FBS. To the lower wells of the chambers, a migration inducing medium (with 10% FBS) was added. The upper wells were filled with a serum-free medium with cells (100,000 cells per well), and the lower chambers were filled with an RPMI 1640 medium supplemented with 10% FBS to induce cell migration. After 24 hours, the assays were stopped by the removal of the medium from the upper wells and the careful removal of the filters. The filters were fixed with methanol and then stained with 20% Giemsa solution (Sigma). The cell number on each filter was counted under a microscope (200X), and 6 fields were randomly selected on each filter for further statistical analysis.

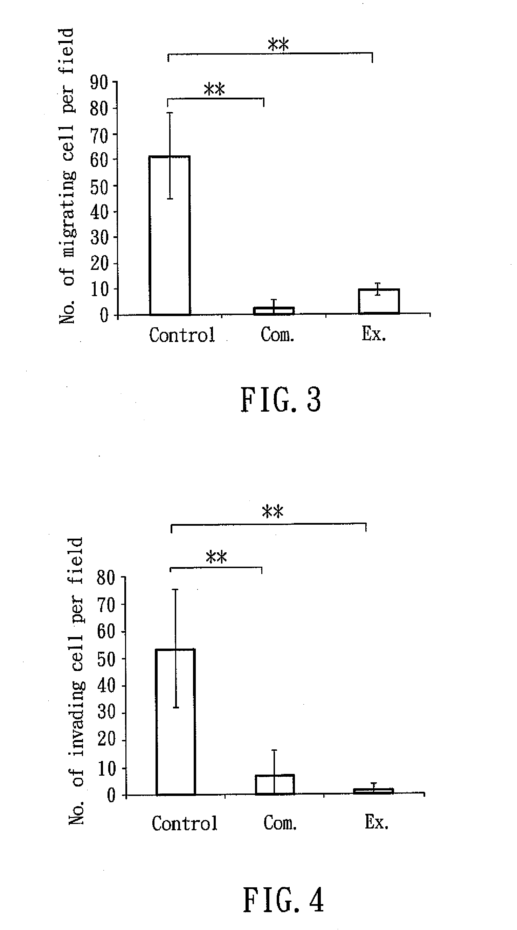

As shown in FIG. 3, a decrease in migration was observed in the SERPINA1-siRNA-transfected CL1-5 cells (experimental group, Ex.) in comparison with the scrambled-siRNA transfected CL1-5 cells (control group, Control) due to RNA interferencing. In addition, a decrease in migration was observed in the FN1-siRNA-transfected CL1-5 cells (comparative group, Comp.).

### Matrigel invasion assay

Cell invasion was examined in a membrane invasion culture system. A transwell membrane (8-µm pore size, BD Biosciences) coated with Matrigel basement membrane matrix (2.5 mg/mL; BD Biosciences Discovery Labware) was used for the invasion assay. Cells (1x10⁵) were seeded into the upper wells in an RPMI 1640 medium, and the lower chambers were filled with an RPMI 1640 medium supplemented with 10% FBS. After incubating at 37°C for 24 h, the membranes were fixed with methanol and the cells were stained with Giemsa staining. The cell number on each filter was counted under a microscope (200X).

The results show that a decrease in invasion was observed in the SERPINA1-siRNA-transfected CL1-5 cells (experimental group, Ex.) in comparison with the scrambled-siRNA transfected CL1-5 cells (control group, Control) due to RNA interference. In addition, a decrease in invasion was observed in the FN1-siRNA-transfected CL1-5 cells (comparative group, Comp.), as shown in FIG. 4.

### Flow cytometry

Fluorescence-activated cell sorting (FACS) was performed to quantify FN1 expression on the cell surfaces. The CL1-0 and CL1-5 cells were trypsinized and incubated in suspension for 2 hrs in 20% FBS media and were washed once with PBS. Cells were incubated with a rabbit anti-FN1 antibody (diluted 1:600 in PBS with 1% BSA, Sigma) for 1 hr at 4°C before they were stained with a fluorescein isothiocyanate-conjugated donkey anti-rabbit antibody in PBS containing 1% BSA for 1 hr at 4°C and fixed in 2% paraformaldehyde in PBS. FACS analysis was performed on a Coulter Epics Profile (FACSCalibur, BD Biosciences, San Jose, California, USA). The nonspecific fluorescence was accounted for by incubating the tumor cells with non-immune serum rather than the primary antibody.

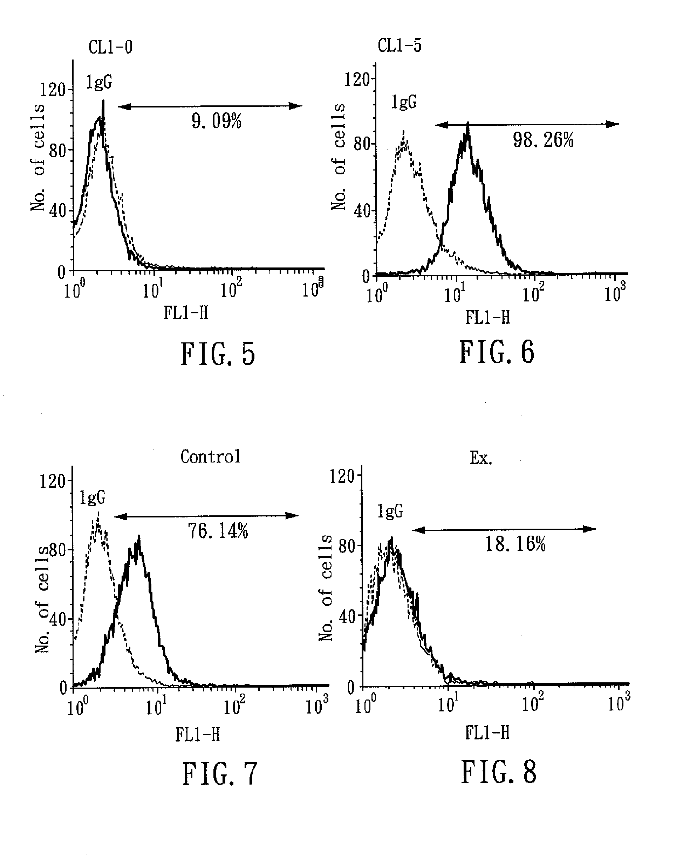

As shown in FIG. 5, the expression of FN1 can be found on surfaces of only 9.09% CL1-0 cells with low invasive capacity. As shown in FIG. 6, the expression of FN1 can be found on surfaces of 98.26% CL1-5 cells with high invasive capacity. As shown in FIG. 7, the expression of FN1 can be found on surface of 76.16% scrambled-siRNA-transfected CL1-5 cells. However, as shown in FIG. 8, the expression of FN1 can be found on surface of only 18.16% SERPINA1-siRNA-transfected CL1-5 cells, and it is because that the expression of SERPINA1 was knockout by SERPINA1 siRNA. These results indicate that SERPINA1 may regulate FN1 aggregating on cell surfaces to inhibit cancer metastasis.

### Lung colony assay

The transfected cells were incubated in suspension for 2 hrs in 20% FBS media. Eight-week-old nude mice were injected in the lateral tail vein with a single-cell suspension that contained 2×10⁶ cells in 0.2 mL RPMI-1640 base medium. The mice were sacrificed after 8 weeks, and the lungs were removed and fixed in 3.7% formalin fixative. The representative lung tumors were removed, fixed, and immediately embedded in paraffin, which was sectioned into 4-mm layers and stained with hematoxylin and eosin (H&E) for histologic analysis.

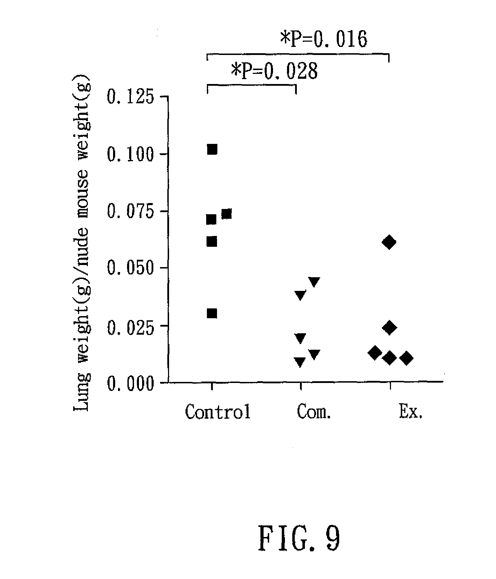

The result of the present experiment is shown in a ratio of lung weight to nude mouse weight. The metastatic tumor cells in lung and the lung weight of the mice treated with SERPINA1-siRNA-transfected CL1-5 cells were fewer and lighter than those treated with scrambled-siRNA-transfected CL1-5 cells, due to the siRNA of SERPINA1. In addition, fewer metastatic tumor cells in lung were also found in the mice treated with FN1-siRNA-transfected CL1-5 cells, as shown in FIG. 9. Furthermore, H&E staining of mouse lungs confirmed that the pulmonary alveoli were filled with metastatic tumor cells in the mice treated with scrambled-siRNA-transfected CL1-5 cells. However, there was a sufficient space in pulmonary alveoli of the mice treated with SERPINA1-siRNA-transfected CL1-5 cells, and only few pulmonary alveoli were filled with metastatic tumor cells. This is because the SERPINA1 siRNA can interfere in the expression of SERPINA1. In addition, there was also a sufficient space in pulmonary alveoli of the mice treated with FN1-siRNA-transfected CL1-5 cells.

In conclusion, both the migration and invasive capacity of CL1-5 lung tumor cells indeed can be reduced by inhibiting the expression of SERPINA1 protein. In addition, the aggregation of FN1 protein on surfaces of tumor cells can also be reduced by inhibiting the expression of SERPINA1 protein. The present invention confirms that SERPINA1 protein is highly related to the invasion/migration of lung tumor cells. Hence, when the RNAi gene therapy of the present invention is applied, the expression of SERPINA1 can be reduced, and therefore the cancer metastasis can further be inhibited.

Although the present invention has been explained in relation to its preferred embodiment, it is to be understood that many other possible modifications and variations can be made without departing from the spirit and scope of the invention as hereinafter claimed.

## Claims

1. A method for evaluating cancer metastasis, comprising following steps:
(A) providing a sample of a subject including a normal tissue and a tissue to be detected;
(B) detecting expression of a biomarker in the normal tissue and the tissue to be detected respectively, wherein the biomarker is serpin peptidase inhibitor, clade A α1-antitrypsin (SERPINA1); and
(C) comparing the expression of the biomarker in the normal tissue with that in the tissue to be detected, wherein when the expression of the biomarker in the normal tissue is higher than that in the tissue to be detected, this indicates that the subject is at a risk of suffering from metastasis.

2. The method as claimed in claim 1, wherein the normal tissue is a normal organ tissue or a normal blood tissue, and the tissue to be detected is a tumor tissue or a blood tissue from a subject with lung cancer.

3. The method as claimed in claim 1 or 2, wherein the expression of mRNA, protein, protein derivatives, or protein fragments of SERPINA1 in the normal tissue or the tissue to be detected are detected respectively in the step (B).

4. The method as claimed in any one of claims 1 to 3, wherein the expression of protein of SERPINA1 in the normal tissue or the tissue to be detected are detected respectively.

5. The method as claimed in claim 4, wherein the expression of protein of SERPINA1 in the normal tissue or the tissue to be detected are detected respectively through western blot analysis, electrophoresis, enzyme-linked immunosorbent assay (ELISA), immunohistochemistry (IHC), immunoprecipitation (IP) or mass spectrometry (MS).

6. The method as claimed in any one of claims 1 to 5, wherein the nucleotide sequence of SERPINA1 has 50% or more identity to a sequence represented by SEQ ID NO: 1.

7. The method as claimed in any one of claims 1 to 6, wherein the amino-acid sequence of SERPINA1 has 50% or more identity to the sequence represented by SEQ ID NO: 2.

8. The method as claimed in any one of claims 2 to 7, wherein the lung cancer is a non-small lung cancer.

9. A biomarker for use in evaluating metastasis, which is at least one selected from a group consisting of a nucleotide sequence, a complementary sequence of the nucleotide sequence, a derivative of the nucleotide sequence, an amino-acid sequence, a derivative of the amino-acid sequence, a fragment of the amino-acid sequence, a mutation of the amino-acid sequence, and an antibody, wherein said nucleotide sequence encodes the amino-acid sequence of SERPINA1, said amino acid sequence is that of SERPINA1 and said antibody binds to SERPINA1.

10. The biomarker as claimed in claim 9, which is at least one selected from the group consisting of the amino-acid sequence, the derivative of the amino-acid sequence, the fragment of the amino-acid sequence, the mutation of the amino-acid sequence, and the antibody.

11. The biomarker as claimed in claim 9 or 10, wherein the nucleotide sequence of SERPINA1 has 50% or more identity to the sequence represented by SEQ ID NO: 1.

12. The biomarker as claimed in claim 9 or 10, wherein the amino-acid sequence of SERPINA1 has 50% or more identity to the sequence represented by SEQ ID NO: 2.

13. An siRNA compound for use in inhibiting lung metastasis, comprising:
a target sequence, which is selected from genes of SERPINA1.

14. The siRNA compound as claimed in claim 13, wherein the target sequence comprises 20-25 continuous nucleotides from SERPINA1.

15. The siRNA compound as claimed in claim 13 or 14, wherein the nucleotide sequence of SERPINA1 is represented by SEQ ID NO: 1.


SEQUENCE LISTING

<110>  National Cheng Kung University

<120>  Method and biomarker for evaluating metastasis, and siRNA
       compound for inhibiting metastasis

<130>  1.114113

<150>  61/530,003
<151>  2011-09-01

<160>  14

<170>  PatentIn version 3.3

<210>  1
<211>  13946
<212>  DNA
<213>  Homo sapiens

<400>  1
   tgggcaggaa ctgggcactg tgcccagggc atgcactgcc tccacgcagc aaccctcaga      60
   gtcctgagct gaaccaagaa ggaggagggg gtcgggcctc cgaggaaggc ctagccgctg     120
   ctgctgccag gaattccagg ttggaggggc ggcaacctcc tgccagcctt caggccactc     180
   tcctgtgcct gccagaagag acagagcttg aggagagctt gaggagagca ggaaaggtgg     240
   gacattgctg ctgctgctca ctcagttcca caggtgggag ggacagcagg gcttagagtg     300
   ggggtcattg tgcagatggg aaaacaaagg cccagagagg ggaagaaatg cccaggagct     360
   accgagggca ggcgacctca accacagccc agtgctggag ctgtgagtgg atgtagagca     420
   gcggaatatc cattcagcca gctcagggga aggacagggg ccctgaagcc aggggatgga     480
   gctgcaggga agggagctca gagagaaggg gaggggagtc tgagctcagt ttcccgctgc     540
   ctgaaaggag ggtggtacct actcccttca cagggtaact gaatgagaga ctgcctggag     600
   gaaagctctt caagtgtggc ccaccccacc ccagtgacac cagcccctga cacgggggag     660
   ggagggcagc atcaggaggg gctttctggg cacacccagt acccgtctct gagctttcct     720
   tgaactgttg cattttaatc ctcacagcag ctcaacaagg tacataccgt caccatcccc     780
   attttacaga tagggaaatt gaggctcgga gcggttaaac aactcacctg aggcctcaca     840
   gccagtaagt gggttccctg gtctgaatgt gtgtgctgga ggatcctgtg ggtcactcgc     900
   ctggtagagc cccaaggtgg aggcataaat gggactggtg aatgacagaa ggggcaaaaa     960
   tgcactcatc cattcactct gcaagtatct acggcacgta cgccagctcc caagcaggtt    1020
   tgcgggttgc acagcgggcg atgcaatctg atttaggctt ttaaagggat tgcaatcaag    1080
   tggggcccca ctagcctcaa ccctgtacct cccctcccct ccacccccag cagtctccaa    1140
   aggcctccaa caaccccaga gtgggggcca tgtatccaaa gaaactccaa gctgtatacg    1200
   gatcacactg gttttccagg agcaaaaaca gaaacaggcc tgaggctggt caaaattgaa    1260
   cctcctcctg ctctgagcag cctggggggc agactaagca gagggctgtg cagacccaca    1320
   taaagagcct actgtgtgcc aggcacttca cccgaggcac ttcacaagca tgcttgggaa    1380
   tgaaacttcc aactctttgg gatgcaggtg aaacagttcc tggttcagag aggtgaagcg 1440
   gcctgcctga ggcagcacag ctcttcttta cagatgtgct tccccacctc taccctgtct 1500
   cacggccccc catgccagcc tgacggttgt gtctgcctca gtcatgctcc atttttccat 1560
   cgggaccatc aagagggtgt ttgtgtctaa ggctgactgg gtaactttgg atgagcggtc 1620
   tctccgctct gagcctgttt cctcatctgt caaatgggct ctaacccact ctgatctccc 1680
   agggcggcag taagtcttca gcatcaggca ttttggggtg actcagtaaa tggtagatct 1740
   tgctaccagt ggaacagcca ctaaggattc tgcagtgaga gcagagggcc agctaagtgg 1800
   tactctccca gagactgtct gactcacgcc accccctcca ccttggacac aggacgctgt 1860
   ggtttctgag ccaggtacaa tgactccttt cggtaagtgc agtggaagct gtacactgcc 1920
   caggcaaagc gtccgggcag cgtaggcggg cgactcagat cccagccagt ggacttagcc 1980
   cctgtttgct cctccgataa ctggggtgac cttggttaat attcaccagc agcctccccc 2040
   gttgcccctc tggatccact gcttaaatac ggacgaggac agggccctgt ctcctcagct 2100
   tcaggcacca ccactgacct gggacagtga atcgtaagta tgcctttcac tgcgagaggt 2160
   tctggagagg cttctgagct ccccatggcc caggcaggca gcaggtctgg ggcaggaggg 2220
   gggttgtgga gtgggtatcc gcctgctgag gtgcagggca gatggagagg ctgcagctga 2280
   gctcctattt tcataataac agcagccatg agggttgtgt cctgtttccc agtcctgccc 2340
   ggtcccccct cggtacctcc tggtggatac actggttcct gtaagcagaa gtggatgagg 2400
   gtgtctaggt ctgcagtcct ggcaccccag gatgggggac accagccaag atacagcaac 2460
   agcaacaaag cgcagccatt tctttctgtt tgcacagctc ctctgtctgt cgggggctcc 2520
   tgtctgttgt ctcctataag cctcaccacc tctcctactg cttgggcatg catctttctc 2580
   cccttctata gatgaggagg ttaaggtcca gagaggggtg gggaggaacg ccggctcaca 2640
   ttctccatcc cctccagata tgaccaggaa cagacctgtg ccaggcctca gccttacatc 2700
   aaaatgggcc tccccatgca ccgtggacct ctgggccctc ctgtcccagt ggaggacagg 2760
   aagctgtgag gggcactgtc acccagggct caagctggca ttcctgaata atcgctctgc 2820
   accaggccac ggctaagctc agtgcgtgat taagcctcat aaccctccaa ggcagttact 2880
   agtgtgattc ccattttaca gatgaggaag atggggacag agaggtgaat aactggcccc 2940
   aaatcacaca ccatccataa ttcgggctca ggcacctggc tccagtcccc aaactcttga 3000
   acctggccct agtgtcactg tttctcttgg gtctcaggcg ctggatgggg aacaggaaac 3060
   ctgggctgga cttgaggcct ctctgatgct cggtgacttc agacagttgc tcaacctctc 3120
   tgttctcttg ggcaaaacat gataaccttt gacttctgtc ccctcccctc accccacccg 3180
   accttgatct ctgaagtgtt ggaaggattt aatttttcct gcactgagtt ttggagacag 3240
   gtcaaaaaga tgaccaaggc caaggtggcc agtttcctat agaacgcctc taaaagacct 3300
   gcagcaatag cagcaagaac tggtattctc gagaacttgc tgcgcagcag gcacttcttg 3360
   gcattttatg tgtatttaat ttcacaatag ctctatgaca aagtccacct ttctcatctc 3420
   caggaaactg aggttcagag aggttaagta acttgtccaa ggtcacacag ctaatagcaa 3480
   gttgacgtgg agcaatctgg cctcagagcc tttaatttta gccacagact gatgctcccc 3540
   tcttcattta gccaggctgc ctctgaagtt ttctgattca agacttctgg cttcagcttt 3600
   gtacacagag atgattcaat gtcaggtttt ggagtgaaat ctgtttaatc ccagacaaaa 3660
   catttaggat tacatctcag ttttgtaagc aagtagctct gtgattttta gtgagttatt 3720
   taatgctctt tggggctcaa tttttctatc tataaaatag ggctaataat ttgcacctta 3780
   tagggtaagc tttgaggaca gattagatga tacggtgcct gtaaaacacc aggtgttagt 3840
   aagtgtggca atgatggtga cgctgaggct gatgtttgct tagcataggg ttaggcagct 3900
   ggcaggcagt aaacagttgg ataatttaat ggaaaatttg ccaaactcag atgctgttca 3960
   ctgctgagca ggagcccctt cctgctgaaa tggtcctggg gagtgcagca ggctctccgg 4020
   gaagaaatct accatctctc gggcaggagc tcaacctgtg tgcaggtaca gggagggctt 4080
   cctcacctgg tgcccactca tgcattacgt cagttattcc tcatccctgt ccaaaggatt 4140
   cttttctcca ttgtacagct atgaagctag tgctcaaaga agtgaagtca tttaccccag 4200
   gccccctgcc agtaagtgac agggcctggt cacacttggg tttatttatt gcccagttca 4260
   acaggttgtt tgaccatagg cgagattctc ttccctgcac cctgccgggt tgctcttggt 4320
   cccttatttt atgctcccgg gtagaaatgg tgtgagatta ggcagggagt ggctcgcttc 4380
   cctgtccctg gccccgcaaa gagtgctccc acctgccccg atcccagaaa tgtcaccatg 4440
   aagccttcat tcttttggtt taaagcttgg cctcagtgtc cgtacaccat ggggtacttg 4500
   gccagatggc gactttctcc tctccagtcg ccctcccagg cactagcttt taggagtgca 4560
   gggtgctgcc tctgatagaa gggccaggag agagcaggtt ttggagtcct gatgttataa 4620
   ggaacagctt gggaggcata atgaacccaa catgatgctt gagaccaatg tcacagccca 4680
   attctgacat tcatcatctg agatctgagg acacagctgt ctcagttcat gatctgagtg 4740
   ctgggaaagc caagacttgt tccagctttg tcactgactt gctgtatagc ctcaacaagg 4800
   ccctgaccct ctctgggctt caaactcttc actgtgaaag gaggaaacca gagtaggtga 4860
   tgtgacacca ggaaagatgg atgggtgtgg gggaatgtgc tcctcccagc tgtcaccccc 4920
   tcgccaccct ccctgcacca gcctctccac ctcctttgag cccagaattc ccctgtctag 4980
   gagggcacct gtctcatgcc tagccatggg aattctccat ctgttttgct acattgaacc 5040
   cagatgccat tctaaccaag aatcctggct gggtgcaggg gctctcgcct gtaaccccag 5100
   cactttggga ggccaaggca ggcggatcaa gaggtcagga gttcaagacc tgcctggcca 5160
   acacggtgaa acctcagctc tactaaaaat acaaaaatta gccaggcgtg gtggcacacg 5220
   cctgtaatcc cagctatttg ggaagctgag acagaagaat ttcttgaacc cgggaggtgg 5280
   aggtttcagt gagccgagat cacgccactg cactccaccc tggcagataa agcgagactc 5340
   tgtctcaaaa aaaacccaaa aacctatgtt agtgtacaga gggccccagt gaagtcttct 5400
   cccagcccca ctttgcacaa ctggggagag tgaggcccca ggaccagagg attcttgcta 5460
   aaggccaagt ggatagtgat ggccctgcca gggctagaag ccacaacctc tggccctgag 5520
   gccactcagc atatttagtg tccccaccct gcagaggccc aactccctcc tgaccactga 5580
   gccctgtaat gatgggggaa tttccataag ccatgaagga ctgcacaaag ttcagttggg 5640
   aagtgaaaga gaaattaaag ggagatggaa atatacagca ctaattttag caccgtcttt 5700
   agttctaaca acactagcta gctgaagaaa aatacaaaca tgtattatgt aatgtgtggt 5760
   ctgttccatt tggattactt agaggcacga gggccaggag aaaggtggtg gagagaaacc 5820
   agctttgcac ttcatttgtt gctttattgg aaggaaactt ttaaaagtcc aagggggttg 5880
   aagaatctca atatttgtta tttccagctt tttttctcca gtttttcatt tcccaaattc 5940
   aaggacacct ttttctttgt attttgttaa gatgatggtt ttggttttgt gactagtagt 6000
   taacaatgtg gctgccgggc atattctcct cagctaggac ctcagttttc ccatctgtga 6060
   agacggcagg ttctacctag ggggctgcag gctggtggtc cgaagcctgg gcatatctgg 6120
   agtagaagga tcactgtggg gcagggcagg ttctgtgttg ctgtggatga cgttgacttt 6180
   gaccattgct cggcagagcc tgctctcgct ggttcagcca caggccccac cactccctat 6240
   tgtctcagcc ccgggtatga aacatgtatt cctcactggc ctatcacctg aagcctttga 6300
   atttgcaaca cctgccaacc cctccctcaa aagagttgcc ctctcagatc cttttgatgt 6360
   aaggtttggt gttgagactt atttcactaa attctcatac ataaacatca ctttatgtat 6420
   gaggcaaaat gaggaccagg gagatgaatg acttgtcctg gctcatacac ctggaaagtg 6480
   acagagtcag attagatccc aggtctatct gaagttaaaa gaggtgtctt ttcacttccc 6540
   acctcctcca tctactttaa agcagcacaa acccctgctt tcaaggagag atgagcgtct 6600
   ctaaagcccc tgacagcaag agcccagaac tgggacacca ttagtgaccc agacggcagg 6660
   taagctgact gcaggagcat cagcctattc ttgtgtctgg gaccacagag cattgtgggg 6720
   acagccccgt ctcttgggaa aaaaacccta agggctgagg atccttgtga gtgttgggtg 6780
   ggaacagctc ccaggaggtt taatcacagc ccctccatgc tctctagctg ttgccattgt 6840
   gcaagatgca tttcccttct gtgcagcagt ttccctggcc actaaatagt gggattagat 6900
   agaagccctc caagggcttc cagcttgaca tgattcttga ttctgatctg gcccgattcc 6960
   tggataatcg tgggcaggcc cattcctctt cttgtgcctc attttcttct tttgtaaaac 7020
   aatggctgta ccatttgcat cttagggtca ttgcagatgt aagtgttgct gtccagagcc 7080
   tgggtgcagg acctagatgt aggattctgg ttctgctact tcctcagtga cattgaatag 7140
   ctgacctaat ctctctggct ttggtttctt catctgtaaa agaaggatat tagcattagc 7200
   acctcacggg attgttacaa gaaagcaatg aattaacaca tgtgagcacg gagaacagtg 7260
   cttggcatat ggtaagcact acgtacattt tgctattctt ctgattcttt cagtgttact 7320
   gatgtcggca agtacttggc acaggctggt ttaataatcc ctaggcactt ccacgtggtg 7380
   tcaatccctg atcactggga gtcatcatgt gccttgactc ggggcctggc ccccccatct 7440
   ctgtcttgca ggacaatgcc gtcttctgtc tcgtggggca tcctcctgct ggcaggcctg 7500
   tgctgcctgg tccctgtctc cctggctgag gatccccagg gagatgctgc ccagaagaca 7560
   gatacatccc accatgatca ggatcaccca accttcaaca agatcacccc caacctggct 7620
   gagttcgcct tcagcctata ccgccagctg gcacaccagt ccaacagcac caatatcttc 7680
   ttctccccag tgagcatcgc tacagccttt gcaatgctct ccctggggac caaggctgac 7740
   actcacgatg aaatcctgga gggcctgaat ttcaacctca cggagattcc ggaggctcag 7800
   atccatgaag gcttccagga actcctccgt accctcaacc agccagacag ccagctccag 7860
   ctgaccaccg gcaatggcct gttcctcagc gagggcctga agctagtgga taagtttttg 7920
   gaggatgtta aaaagttgta ccactcagaa gccttcactg tcaacttcgg ggacaccgaa 7980
   gaggccaaga aacagatcaa cgattacgtg gagaagggta ctcaagggaa aattgtggat 8040
   ttggtcaagg agcttgacag agacacagtt tttgctctgg tgaattacat cttctttaaa 8100
   ggtaaggttg ctcaaccagc ctgagctgtt cccatagaaa caagcaaaaa tattctcaaa 8160
   ccatcagttc ttgaactctc cttggcaatg cattatgggc catagcaatg cttttcagcg 8220
   tggattcttc agttttctac acacaaacac taaaatgttt tccatcattg agtaatttga 8280
   ggaaataata gattaaactg tcaaaactac tgacagctct gcagaacttt tcagagcctt 8340
   taatgtcctt gtgtatactg tatatgtaga atatataatg cttagaacta tagaacaaat 8400
   tgtaatacac tgcataaagg gatagtttca tggaacatac tttacacgac tctagtgtcc 8460
   cagaatcagt atcagttttg caatctgaaa gacctgggtt caaatcctgc ctctaacaca 8520
   attagctttt gacaaaaaca atgcattcta cctctttgag gtgctaattt ctcatcttag 8580
   catggacaaa ataccattct tgctgtcagg tttttttagg attaaacaaa tgacaaagac 8640
   tgtggggatg gtgtgtggca tacagcaggt gatggactct tctgtatctc aggctgcctt 8700
   cctgcccctg aggggttaaa atgccagggt cctgggggcc ccagggcatt ctaagccagc 8760
   tcccactgtc ccaggaaaac agcatagggg aggggaggtg ggaggcaagg ccaggggctg 8820
   cttcctccac tctgaggctc ccttgctctt gaggcaaagg agggcagtgg agagcagcca 8880
   ggctgcagtc agcacagcta aagtcctggc tctgctgtgg ccttagtggg ggcccaggtc 8940
   cctctccagc cccagtctcc tccttctgtc caatgagaaa gctgggatca ggggtccctg 9000
   aggcccctgt ccactctgca tgcctcgatg gtgaagctct gttggtatgg cagaggggag 9060
   gctgctcagg catctgcatt tcccctgcca atctagagga tgaggaaagc tctcaggaat 9120
   agtaagcaga atgtttgccc tggatgaata actgagctgc caattaacaa ggggcaggga 9180
   gccttagaca gaaggtacca aatatgcctg atgctccaac attttatttg taatatccaa 9240
   gacaccctca aataaacata tgattccaat aaaaatgcac agccacgatg gcatctctta 9300
   gcctgacatc gccacgatgt agaaattctg catcttcctc tagttttgaa ttatccccac 9360
   acaatctttt tcggcagctt ggatggtcag tttcagcacc ttttacagat gatgaagctg 9420
   agcctcgagg gatgtgtgtc gtcaaggggg ctcagggctt ctcagggagg ggactcatgg 9480
   tttctttatt ctgctacact cttccaaacc ttcactcacc cctggtgatg cccaccttcc 9540
   cctctctcca ggcaaatggg agagaccctt tgaagtcaag gacaccgagg aagaggactt 9600
   ccacgtggac caggtgacca ccgtgaaggt gcctatgatg aagcgtttag gcatgtttaa 9660
   catccagcac tgtaagaagc tgtccagctg ggtgctgctg atgaaatacc tgggcaatgc 9720
   caccgccatc ttcttcctgc ctgatgaggg gaaactacag cacctggaaa atgaactcac 9780
   ccacgatatc atcaccaagt tcctggaaaa tgaagacaga aggtgattcc ccaacctgag 9840
   ggtgaccaag aagctgccca cacctcttag ccatgttggg actgaggccc atcaggactg 9900
   gccagagggc tgaggagggt gaaccccaca tccctgggtc actgctactc tgtataaact 9960
   tggcttccag aatgaggcca ccactgagtt caggcagcgc catccatgct ccatgaggag 10020
   gacagtaccc aggggtgagg aggtaaaggt ctcgtccctg gggacttccc actccagtgt 10080
   ggacactgtc ccttcccaat atccagtgcc cagggcaggg acagcagcac caccacacgt 10140
   tctggcagaa ccaaaaagga acagatgggc ttcctggcaa aggcagcagt ggagtgtgga 10200
   gttcaagggt agaatgtccc tggggggacg ggggaagagc ctgtgtggca aggcccagaa 10260
   aagcaaggtt cggaattgga acagccaggc catgttcgca gaaggcttgc gtttctctgt 10320
   cactttatcg gtgctgttag attgggtgtc ctgtagtaag tgatacttaa acatgagcca 10380
   cacattagtg tatgtgtgtg cattcgtgat tatgcccatg ccctgctgat ctagttcgtt 10440
   ttgtacactg taaaaccaag atgaaaatac aaaaggtgtc gggttcataa taggaatcga 10500
   ggctggaatt tctctgttcc atgccagcac ctcctgaggt ctctgctcca ggggttgaga 10560
   aagaacaaag aggctgagag ggtaacggat cagagagccc agagccaagc tgcccgctca 10620
   caccagaccc tgctcagggt ggcattgtct ccccatggaa aaccagagag gagcactcag 10680
   cctggtgtgg tcactcttct cttatccact aaacggttgt cactgggcac tgccaccagc 10740
   cccgtgtttc tctgggtgta gggccctggg gatgttacag gctgggggcc aggtgaccca 10800
   acactacagg gcaagatgag acaggcttcc aggacaccta gaatatcaga ggaggtggca 10860
   tttcaagctt ttgtgattca ttcgatgtta acattctttg actcaatgta gaagagctaa 10920
   aagtagaaca aaccaaagcc gagttcccat cttagtgtgg gtggaggaca caggagtaag 10980
   tggcagaaat aatcagaaaa gaaaacactt gcactgtggt gggtcccaga agaacaagag 11040
   gaatgctgtg ccatgccttg aatttctttt ctgcacgaca ggtctgccag cttacattta 11100
   cccaaactgt ccattactgg aacctatgat ctgaagagcg tcctgggtca actgggcatc 11160
   actaaggtct tcagcaatgg ggctgacctc tccggggtca cagaggaggc acccctgaag 11220
   ctctccaagg tgagatcacc ctgacgacct tgttgcaccc tggtatctgt agggaagaat 11280
   gtgtgggggc tgcagctctg tcctgaggct gaggaagggg ccgagggaaa caaatgaaga 11340
   cccaggctga gctcctgaag atgcccgtga ttcactgaca cgggacgtgg tcaaacagca 11400
   aagccaggca ggggactgct gtgcagctgg cactttcggg gcctcccttg aggttgtgtc 11460
   actgaccctg aatttcaact ttgcccaaga ccttctagac attgggcctt gatttatcca 11520
   tactgacaca gaaaggtttg ggctaagttg tttcaaagga atttctgact ccttcgatct 11580
   gtgagatttg gtgtctgaat taatgaatga tttcagctaa agatgacact tattttggaa 11640
   aactaaaggc gaccaatgaa caactgcagt tccatgaatg gctgcattat cttggggtct 11700
   gggcactgtg aaggtcactg ccagggtccg tgtcctcaag gagcttcaag ccgtgtacta 11760
   gaaaggagag agccctggag gcagacgtgg agtgacgatg ctcttccctg ttctgagttg 11820
   tgggtgcacc tgagcagggg gagaggcgct tgtcaggaag atggacagag gggagccagc 11880
   cccatcagcc aaagccttga ggaggagcaa ggcctatgtg acagggaggg agaggatgtg 11940
   cagggccagg gccgtccagg gggagtgagc gcttcctggg aggtgtccac gtgagccttg 12000
   ctcgaggcct gggatcagcc ttacaacgtg tctctgcttc tctcccctcc aggccgtgca 12060
   taaggctgtg ctgaccatcg acgagaaagg gactgaagct gctggggcca tgtttttaga 12120
   ggccataccc atgtctatcc cccccgaggt caagttcaac aaaccctttg tcttcttaat 12180
   gattgaacaa aataccaagt ctcccctctt catgggaaaa gtggtgaatc ccacccaaaa 12240
   ataactgcct ctcgctcctc aacccctccc ctccatccct ggccccctcc ctggatgaca 12300
   ttaaagaagg gttgagctgg tccctgcctg catgtgactg taaatccctc ccatgttttc 12360
   tctgagtctc cctttgcctg ctgaggctgt atgtgggctc caggtaacag tgctgtcttc 12420
   gggccccctg aactgtgttc atggagcatc tggctgggta ggcacatgct gggcttgaat 12480
   ccagggggga ctgaatcctc agcttacgga cctgggccca tctgtttctg gagggctcca 12540
   gtcttccttg tcctgtcttg gagtccccaa gaaggaatca caggggagga accagatacc 12600
   agccatgacc ccaggctcca ccaagcatct tcatgtcccc ctgctcatcc cccactcccc 12660
   cccacccaga gttgctcatc ctgccagggc tggctgtgcc caccccaagg ctgccctcct 12720
   gggggcccca gaactgcctg atcgtgccgt ggcccagttt tgtggcatct gcagcaacac 12780
   aagagagagg acaatgtcct cctcttgacc cgctgtcacc taaccagact cgggccctgc 12840
   acctctcagg cacttctgga aaatgactga ggcagattct tcctgaagcc cattctccat 12900
   ggggcaacaa ggacacctat tctgtccttg tccttccatc gctgccccag aaagcctcac 12960
   atatctccgt ttagaatcag gtcccttctc cccagatgaa gaggagggtc tctgctttgt 13020
   tttctctatc tcctcctcag acttgaccag gcccagcagg ccccagaaga ccattaccct 13080
   atatcccttc tcctccctag tcacatggcc ataggcctgc tgatggctca ggaaggccat 13140
   tgcaaggact cctcagctat gggagaggaa gcacatcacc cattgacccc cgcaacccct 13200
   ccctttcctc ctctgagtcc cgactggggc cacatgcagc ctgacttctt tgtgcctgtt 13260
   gctgtccctg cagtcttcag agggccaccg cagctccagt gccacggcag gaggctgttc 13320
   ctgaatagcc cctgtggtaa gggccaggag agtccttcca tcctccaagg ccctgctaaa 13380
   ggacacagca gccaggaagt cccctgggcc cctagctgaa ggacagcctg ctccctccgt 13440
   ctctaccagg aatggccttg tcctatggaa ggcactgccc catcccaaac taatctagga 13500
   atcactgtct aaccactcac tgtcatgaat gtgtacttaa aggatgaggt tgagtcatac 13560
   caaatagtga tttcgatagt tcaaaatggt gaaattagca attctacatg attcagtcta 13620
   atcaatggat accgactgtt tcccacacaa gtctcctgtt ctcttaagct tactcactga 13680
   cagcctttca ctctccacaa atacattaaa gatatggcca tcaccaagcc ccctaggatg 13740
   acaccagacc tgagagtctg aagacctgga tccaagttct gacttttccc cctgacagct 13800
   gtgtgacctt cgtgaagtcg ccaaacctct ctgagcccca gtcattgcta gtaagacctg 13860
   cctttgagtt ggtatgatgt tcaagttaga taacaaaatg tttataccca ttagaacaga 13920
   gaataaatag aactacattt cttgca 13946

<210> 2
<211> 418
<212> PRT
<213> Homo sapiens

<400> 2

<210>  3
<211>  21
<212>  DNA
<213>  Artificial Sequence

<220>
<223>  SERPINA1 siRNA

<400>  3
ccaacagcac caauaucuut t                                                21

<210>  4
<211>  21
<212>  DNA
<213>  Artificial Sequence

<220>
<223>  SERPINA1 siRNA

<400>  4
aagauauugg ugcuguuggt t                                                21

<210>  5
<211>  21
<212>  DNA
<213>  Artificial Sequence

<220>
<223>  SERPINA1 siRNA

<400>  5
guccauuacu ggaaccuaut t                                                21

<210>  6
<211>  21
<212>  DNA
<213>  Artificial Sequence

<220>
<223>  SERPINA1 siRNA

<400>  6
auagguucca guaauggact t                                                21

<210>  7
<211>  21
<212>  DNA
<213>  Artificial Sequence

<220>
<223>  SERPINA1 siRNA

<400>  7
cgaggucaag uucaacaaat t                                                21

<210>  8
<211>  21
<212>  DNA
<213>  Artificial Sequence

<220>
<223>  SERPINA1 siRNA

<400>  8
uuuguugaac uugaccucgt t                                                21

<210>  9
<211>  21
<212>  DNA
<213>  Artificial Sequence

<220>
<223>  FN1 siRNA

<400>  9
gacugguggu uacauguuat t                                                21


<210>  10
<211>  21
<212>  DNA
<213>  Artificial Sequence

<220>
<223>  FN1 siRNA

<400>  10
uaacauguaa ccaccaguct t                                                21


<210>  11
<211>  21
<212>  DNA
<213>  Artificial Sequence

<220>
<223>  FN1 siRNA

<400>  11
cgcaucacuu gcacuucuat t                                                21


<210>  12
<211>  21
<212>  DNA
<213>  Artificial Sequence

<220>
<223>  FN1 siRNA

<400>  12
uagaagugca agugaugcgt t                                                21


<210>  13
<211>  21
<212>  DNA
<213>  Artificial Sequence

<220>
<223>  FN1 siRNA

<400>  13
gauccugucu acuucacaat t                                                21


<210>  14
<211>  21
<212>  DNA
<213>  Artificial Sequence

<220>
<223>  FN1 siRNA

<400>  14
uugugaagua gacaggauct t                                                21